(19) **Europäisches Patentamt**

**European Patent Office**

**Office européen des brevets**

(11) **EP 0 805 415 B1**

(12) **EUROPÄISCHE PATENTSCHRIFT**

(45) Veröffentlichungstag und Bekanntmachung des Hinweises auf die Patenterteilung:
**09.07.2003 Patentblatt 2003/28**

(51) Int Cl.$^7$: **G06T 5/00**, A61B 6/00

(21) Anmeldenummer: **97201015.1**

(22) Anmeldetag: **07.04.1997**

(54) **Verfahren zur Detektion und Korrektur von Bildverzerrungen bei der medizinischen Bildgebung**

Method of detection and correction of image distortions in medical imaging

Procédé pour la détection et correction de distortions d'image dans l'imagerie médicale

(84) Benannte Vertragsstaaten:
**DE FR GB NL**

(30) Priorität: **19.04.1996 DE 19615456**

(43) Veröffentlichungstag der Anmeldung:
**05.11.1997 Patentblatt 1997/45**

(73) Patentinhaber:
• **Philips Intellectual Property & Standards GmbH
20099 Hamburg (DE)**
Benannte Vertragsstaaten:
**DE**
• **Koninklijke Philips Electronics N.V.
5621 BA Eindhoven (NL)**
Benannte Vertragsstaaten:
**FR GB NL**

(72) Erfinder:
• **Wischmann, Hans-Aloys, Dr.
Röntgenstrasse 24, 22335 Hamburg (DE)**
• **Zylka, Waldemar, Dr.
Röntgenstrasse 24, 22335 Hamburg (DE)**

(74) Vertreter: **Volmer, Georg, Dipl.-Ing. et al
Philips Intellectual Property & Standards GmbH,
Postfach 50 04 42
52088 Aachen (DE)**

(56) Entgegenhaltungen:
**EP-A- 0 422 396            US-A- 5 005 578**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren nach dem Oberbegriff des Anspruchs 1 bei dem mehrere Bilder erstellt werden. Die Erfindung betrifft außerdem eine Computertomographie -Anordnung (im folgenden mit CT-Anordnung bezeichnet) mit einer Röntgendetektoranordnung, einer Recheneinheit zur Verarbeitung gemessener Signale und zur Berechnung von Schichtbildern aus den gemessenen Signalen zur Durchführung eines derartigen Verfahrens

Ein Verfahren zur Korrektur von Bildverzerrungen bei der Computertomographie ist beispielsweise aus der EP-A2-479 618 bekannt. Dort werden mit Hilfe eines Kalibrierobjektes sogenannte geometrische Verzerrungen (Verzerrungen aufgrund der besonderen geometrischen Konstruktion des Bildverstärkers) und magnetische Verzerrungen (durch das Magnetfeld der Erde hervorgerufene Verzerrungen) korrigiert. Das Kalibrierobjekt wird auf die Oberfläche des Bildverstärkers gelegt, von Röntgenstrahlung durchstrahlt, und dadurch wird ein Schichtbild dieses Kalibrierobjektes aufgenommen. Das Kalibrierobjekt ist dabei ein aus Kupferdrähten bestehendes Gitter. Aus dem Schichtbild des Kalibrierobjektes werden eine horizontale und eine vertikale Korrekturtabelle erstellt, die die genannten Verzerrungen beschreiben und später zur Korrektur von CT-Bildern verwendet werden. CT-Bilder können beispielsweise senkrecht oder schräg unter einem Winkel zu den Schichtbildern liegende Bilder sein, die aus in verschiedenen Schichtbildern liegenden Meßwerten gewonnen werden.

Mit dem bekannten Verfahren werden allerdings nur Bildverzerrungen detektiert, die zeitlich vor der Durchstrahlung eines Patienten erfolgen. Auch nur solche Bildverzerrungen können später in den Bildern korrigiert werden. Eine Berücksichtigung von Verzerrungen unmittelbar während der Aufnahmesituation erfolgt dabei nicht. Es ist jedoch durchaus denkbar, daß trotz sorgfältiger mechanischer Kalibrierung des Computertomographen bzw. des sonstigen bildgebenden Systems in den Bildern Verzerrungen auftreten können. Gerade die Anwendung der Computertomographie während operativer Maßnahmen, z.B. in der Neurochirurgie, stellt höchste Anforderungen an die Präzision, um z.B. die exakte Lage eines Tumors im Gehirn feststellen zu können. Deshalb ist die Detektion möglicher Verzerrungen der Bilder und deren Korrektur unbedingt notwendig.

Nachfolgend werden einige Arten von Verzerrungen und ihre möglichen Ursachen genannt. Aufgrund ungenauer Kalibrierung kann eine Winkelfehlstellung zwischen der Durchstrahlungsebene und der Patimentischebene während der Aufnahme auftreten. Meist werden bei CT senkrecht zur Körperlängsachse eines Patienten liegende Schichtebenen aufgenommen, wobei dann die Durchstrahlungsebene senkrecht zur Patientischebene angeordnet sein sollte. Eine Einstellungsungenauigkeit von ± 2° von einem Sollwinkel von 90° ist hierbei allerdings durchaus typisch. Eine solche Fehlstellung führt zu einer scherungsartigen Verzerrung des Bildes.

Eine weitere Verzerrungsart ist die Skalenverzerrung im Bild, die durch fehlende Übereinstimmung zwischen der tatsächlichen und der vorgesehenen Vorschubgeschwindigkeit des Patiententisches auftreten kann. Dadurch hervorgerufene Verzerrungen führen zu gedehnten oder gestauchten Abbildungen des abzubildenden Bereichs.

Das Gewicht des Patienten kann zu einer Durchbiegung des Patiententisches führen, wodurch sowohl eine Winkelfehlstellung zwischen Durchstrahlungsebene und Patientisch als auch ein weiterer Verzerrungseffekt, der im allgemeinen durch eine quadratische Gleichung beschrieben werden kann, auftreten. Außerdem ist eine gegebenenfalls auftretende Rotation und eine translatorische Verschiebung zwischen dem Koordinatensystem, in dem sich der (reale) Phantomkörper befindet, und dem Bild-Koordinatensystem, in dem sich das Bild des Phantomkörpers (das Phantombild) befindet, zu berücksichtigen und zu korrigieren.

Der Erfindung liegt die Aufgabe zugrunde, ein Verfahren der eingangs genannten Art vorteilhaft weiterzubilden. Der Erfindung liegt außerdem die Aufgabe zugrunde, eine CT-Anordnung zur Detektion und Korrektur von Bildverzerrungen in vorteilhafter Weise auszugestalten.

Die erstgenannte Aufgabe wird durch die im Anspruch 1 angegebenen Maßnahmen gelöst. Da der Phantomkörper während der Erstellung der Bilder im Abbildungsbereich angeordnet wird, können unmittelbar dabei auftretende Verzerrungen detektiert und korrigiert werden. In jedem Bild erscheinen Bildpunkte des Phantomkörpers. Mit Bildpunkten sind dabei Punkte gemeint, an denen in den Bildern Punkte des Phantomkörpers abgebildet sind. Aus einer Anzahl solcher Bildpunkte in verschiedenen Bildern kann beispielsweise leicht ein sogenanntes Phantombild, d.h. eine Abbildung des Phantomkörpers konstruiert werden. Aus einem Vergleich dieses Phantombildes mit dem realen Phantomkörper, dessen Struktur und dessen Koordinaten im Raum bekannt sind, wird dann ermittelt, welche Verzerrungen während der Erstellung der Bilder aufgetreten sind und wie sich diese Verzerrungen ausgewirkt haben. Aus diesem Vergleich läßt sich auch eine Korrekturvorschrift herleiten, die die aufgetretenen Verzerrungen beschreibt und nach der die erstellten Bilder korrigiert werden können. Dies führt letzlich zu einer deutlich erhöhten Genauigkeit der Bilder, so daß auch Positionen im Bild, an denen sich z.B. ein Tumor befindet, mit großer Genauigkeit in Positionen im Patienten umgerechnet werden können.

Es sei an dieser Stelle erwähnt, daß aus der US-PS 5,005,578 bereits ein Verfahren bekannt ist, bei dem Verzerrungen in einem MR-Bild mit Hilfe eines Phantomkörper beseitigt werden, der in einem während einer Untersuchung von einem Patienten getragenen Helm untergebracht ist, sodaß er sich oberhalb des Kopfes des Patienten befindet.

Bei der Weiterbildung nach Anspruch 2 muß der Phantomkörper mehrere Elemente enthalten, welche in verschiedenen Ebenen liegen, um in verschiedenen Raumrichtungen auftretende Verzerrungen anhand der Abbildung des Phantomkörpers in den Bildern ermitteln zu können.

Eine bevorzugte Ausgestaltung der Erfindung sieht vor, daß als Phantomkörper ein zwei X-förmige X-Elemente enthaltender X-Rahmen verwendet wird und daß der Phantomkörper derart angeordnet wird, daß die Ebenen, in denen die X-Elemente angeordnet sind, senkrecht zu den in den Bildern abgebildeten Ebenen liegen. Das als X-Element bezeichnete Element weist die Gestalt einer Sanduhr auf und besteht beispielsweise aus vier Stäben, wobei zwei Stäbe parallel zueinander, die beiden anderen Stäbe über kreuz angeordnet sind und diagonal gegenüberliegende Endpunkte der beiden parallelen Stäbe verbinden. Die beiden X-Elemente sind in einem festen Winkel von vorzugsweise exakt 90° zueinander angeordnet und können einen der parallelen Stäbe gemeinsam haben so, daß sich ein zusammenhängender Phantomkörper ergibt. Der gesamte X-Rahmen ist aus einem Material hergestellt, das die Röntgenstrahlung absorbiert, so daß sich in den Bildern Bildpunkte des X-Rahmens zeigen. In jedem Bild werden mit einem derartigen zusammenhängenden X-Rahmen im allgemeinen sieben Bildpunkte abgebildet.

Eine alternative Weiterbildung der Erfindung sieht vor, daß als Phantomkörper ein drei N-förmige N-Elemente enthaltender N-Rahmen verwendet wird, bei dem zwei N-Elemente parallel zueinander und senkrecht zum dritten N-Element angeordnet sind, und daß der Phantomkörper derart angeordnet wird, daß die Ebenen, in denen die N-Elemente angeordnet sind, senkrecht zu den in den Bildern abgebildeten Ebenen liegen. Jedes N-Element kann dabei beispielsweise aus drei Metallstäben aufgebaut sein, wobei zwei Metallstäbe parallel zueinander und der dritte Metallstab zwei Endpunkte dieser beiden Metallstäbe diagonal miteinander verbindet. Zwei benachbarte N-Elemente können auch einen Metallstab gemeinsam haben so, daß der N-Rahmen ein kompakter zusammenhängender Phantomkörper ist. Der gesamte N-Rahmen ist ebenfalls aus einem Material hergestellt, das die Röntgenstrahlung absorbiert, so daß sich in den Bildern Bildpunkte des N-Rahmens zeigen. Bei einem aus drei zusammenhängenden N-Elementen bestehenden N-Rahmen ergeben sich ebenfalls in jedem Bild sieben Bildpunkte des N-Rahmens.

Vorzugsweise sind sowohl beim X-Rahmen als auch beim N-Rahmen die einzelnen Elemente unter einem Winkel von 90° zueinander angeordnet, wodurch sich die höchste Genauigkeit bei der Detektion von Verzerrungen erzielen läßt. Es ist jedoch auch möglich, die Elemente unter einem anderen Winkel (ungleich 0°) zueinander anzuordnen. Dieser Winkel muß jedoch bekannt und während der Erstellung der Bilder fest sein.

Ein Vorteil des X-Rahmens gegenüber dem N-Rahmen liegt darin, daß der X-Rahmen nur aus zwei Elementen besteht, während der N-Rahmen aus drei Elementen besteht. Deshalb erlaubt der X-Rahmen eine deutlich einfachere, flexiblere und freiere Patientenpositionierung beispielsweise auf einem Patientisch. Außerdem kann bei Verwendung eines X-Rahmens das Field-of-View des bildgebenden Systems, beispielsweise des CT-Scanners bei der Computertomographie kleiner gewählt werden, während bei Verwendung eines N-Rahmens Bildpunkte von beiden parallel zueinander angeordneten N-Elementen in den Bildern liegen müssen. Beim N-Rahmen sind auch die möglichen Fehlerquellen für mechanische Ungenauigkeiten größer.

In einer erfindungsgemäßen Weiterbildung des Verfahrens ist vorgesehen, daß die Bildkoordinaten von mindestens fünf charakteristischen Bildpunkten des Phantomkörpers bestimmt werden und daß aus den Bildkoordinaten und den Koordinaten der korrespondierenden charakteristischen Punkte des Phantomkörpers im Raum die Korrekturvorschrift berechnet wird. Zur Ermittlung des Phantombildes werden vorteilhaft eine Reihe von charakteristischen Punkten des Phantomkörpers benutzt. Diese charakteristischen Punkte müssen nicht notwendigerweise direkt in einem Bild abgebildet sein, sie sollten sich lediglich aus anderen in den Bildern erscheinenden Bildpunkten des Phantomkörpers eindeutig bestimmen und den korrespondietenden charakteristischen Punkten des (realen) Phantomkörpers zuordnen lassen. Zur Berechnung einer die obengenannten Verzerrungen sowie eine Translation und eine Rotation zwischen den Koordinatensystemen beschreibenden Korrekturvorschrift durch ein Gleichungssystem müssen die Bildkoordinaten von mindestens fünf charakteristischen Bildpunkten bestimmt werden, da für eine solche Korrekturvorschrift im allgemeinen 13 Unbekannte zu bestimmen sind.

Nach einer weitergehenden Ausgestaltung der Erfindung ist vorgesehen, daß als charakteristische Punkte die Eckpunkte des N-Rahmens bzw. des X-Rahmens ausgewählt werden. Diese Punkte eignen sich besonders, da sie sich jeweils als Schnittpunkt zweier Linien ergeben. Diese Linien ergeben sich durch die Verbindung von Bildpunkten des Phantomkörpers in den einzelnen Bildern. Sowohl bei einem X-Rahmen als auch bei einem N-Rahmen mit jeweils zusammenhängenden Elementen ergeben sich auf diese Weise jeweils 6 Eckpunkte. Bei einem X-Rahmen könnten außerdem auch die Schnittpunkte zweier diagonaler Stäbe eines X-Elements als charakteristische Punkte verwendet werden. Theoretisch können beliebige Punkte ausgewählt werden. Jedoch muß die Zuordnung von Bildpunkt zu zugehörigem Punkt am realen Phantomkörper eindeutig möglich sein. Außerdem ist die Genauigkeit bei Verwendung der Eckpunkte als charakteristische Punkte am größten.

In einer vorteilhaften Weiterbildung der Erfindung ist vorgesehen, daß der Phantomkörper mit einem Patiententisch verbunden ist. Auf diesem Patiententisch ist während der Erstellung der Bilder auch der Patient angeordnet. Der Vorteil dieser Weiterbildung liegt darin, daß damit insbesondere durch Fehlstellung des Patiententisches oder durch falsche Vorschubgeschwindigkeit beim Verschieben des Patiententisches beispielsweise bei CT verursachte Verzerrungen detektiert werden können. Der Patient und der Phantomkörper sind während der Erstellung der Bilder fest zueinander angeordnet, so daß angenommen werden kann, daß die abgebildeten Teile des Patienten und des Phantomkörpers in gleicher Weise verzerrt in den Bildern erscheinen.

In einer davon ausgehenden Weiterbildung der Erfindung ist vorgesehen, daß mit Hilfe von am Phantomkörper in zur Verschiebungsrichtung des Patiententisches paralleler Richtung angebrachten Markierungen Veränderungen der Geschwindigkeit des Patiententisches relativ zum Abbildungsbereich während der Erstellung der Bilder detektiert werden. Die Markierungen werden ebenfalls in den Bildern abgebildet. Die Abstände der Markierungen am Phantomkörper sind bekannt und werden mit den Abständen der Abbildungen der Markierungen in den Bildern verglichen, woraus sich leicht ermitteln läßt, ob die Geschwindigkeit des Patiententisches während der Durchstrahlung wie gewünscht konstant war oder ob sich die Geschwindigkeit veränderte, der Patiententisch also beschleunigt oder abgebremst (oder beides nacheinander) wurde. Dadurch lassen sich dann die Bilder korrigieren. Der Patiententisch könnte auch fest angeordnet sein, wobei dann das bildgebende System, z.B. der CT-Scanner mit vorgegebener Geschwindigkeit gegenüber dem Patiententisch bewegt wird.

Die Erfindung wird insbesondere bei der Computertomographie, bei der die Bilder als Schichtbilder erstellt werden, verwendet. In einer Ausgestaltung der Erfindung ist dabei vorgesehen, daß aus mehreren Schichtbildern ein CT-Bild und aus den Bildpunkten des Phantomkörpers in mehreren Schichtbildern ein Gesamtbild (Phantombild) des Phantomkörpers ermittelt wird.

Erfindungsgemäß ist weiter in einer Ausgestaltung vorgesehen, daß die Korrekturvorschrift durch Einsetzen einer Anzahl von Bildpunkten des Phantomkörpers im Phantombild und den korrespondierenden Punkten des Phantomkörpers im Raum in eine zwischen Bildpunkten und Punkten im Raum bestehende lineare Gleichung ermittelt wird und daß danach eine Korrektur der Bildverzerrungen durch Einsetzen weiterer Bildpunkte des CT-Bildes in diese Gleichung erfolgen kann.

Anspruch 12 beschreibt eine CT-Anordnung zur Durchführung des Verfahrens nach Anspruch 1 und Anspruch 13 eine Weiterbildung dieser CT-Anordnung.

Die Erfindung wird nachfolgend anhand der Zeichnungen näher erläutert. Es zeigen:

Fig. 1    ein Blockschaltbild einer CT-Anordnung, mit dem die Erfindung verwirklicht werden kann,

Fig. 2    eine räumliche Darstellung einer solchen CT-Anordnung,

Fig. 3    ein erfindungsgemäßer Phantomkörper (X-Rahmen),

Fig. 4    eine weitere Ausführung eines erfindungsgemäßen Phantomkörpers (N-Rahmen),

Fig. 5    einen Ablaufplan zur Darstellung der einzelnen Schritte des erfindungsgemäßen Verfahrens und

Fig. 6    ein einzelnes Element (X-Element) des Phantomkörpers gemäß Fig. 3 zur Verdeutlichung des erfindungsgemäßen Verfahrens.

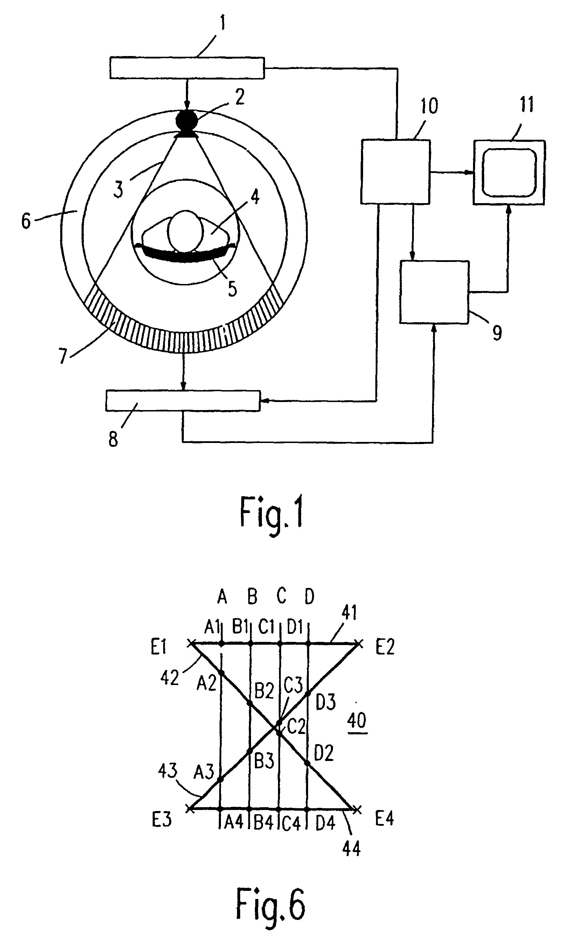

Mit 1 ist in Fig. 1 ein Röntgengenerator bezeichnet. Mit einer Röntgenröhre 2 wird in der CT-Scannereinheit 6 ein fächerförmiges Röntgenstrahlenbündel 3 erzeugt, das den Durchstrahlungsbereich darstellt. In diesem Durchstrahlungsbereich 3 (= Abbildungsbereich) befindet sich ein Patient 4, der auf einem Patiententisch 5 während der Untersuchung in zur Zeichenebene senkrechter Richtung durch das Röntgenstrahlenbündel 3 bewegt wird. Gegenüber der Röntgenröhre 2 ist in der CT-Scannereinheit 6 eine Detektoranordnung 7 mit einer Anzahl von Detektoren angeordnet, die die durch den Patienten 4 geschwächte Röntgenstrahlung erfassen. Während der Durchstrahlung rotiert die CT-Scannereinheit 6 zusammen mit der Röntgenröhre 2 und der Detektoranordnung 7 um den Patienten 4. Dabei wird die abzubildende Körperquerschicht des Patienten 4 mit dem eng begrenzten Röntgenstrahlenbündel 3 in zahlreichen, in einer Ebene liegenden Projektionsrichtungen durchstrahlt. Die von der Detektoranordnung 7 gemessenen Werte werden von einer Datenerfassungseinheit 8 erfaßt und in Digitalwerte gewandelt. Diese Digitalwerte werden an eine Recheneinheit 9 weitergegeben, die daraus einzelne Schichtbilder und ein dreidimensionales CT-Gesamtbild berechnet. Die einzelnen Bilder können auf einem Monitor 11 dargestellt werden. Die gesamte CT-Anordnung wird von einer Steuereinheit 10 gesteuert. Weitere Elemente, wie beispielsweise eine Bedieneinheit oder eine Speichereinheit, sind der Übersichtlichkeit halber weggelassen.

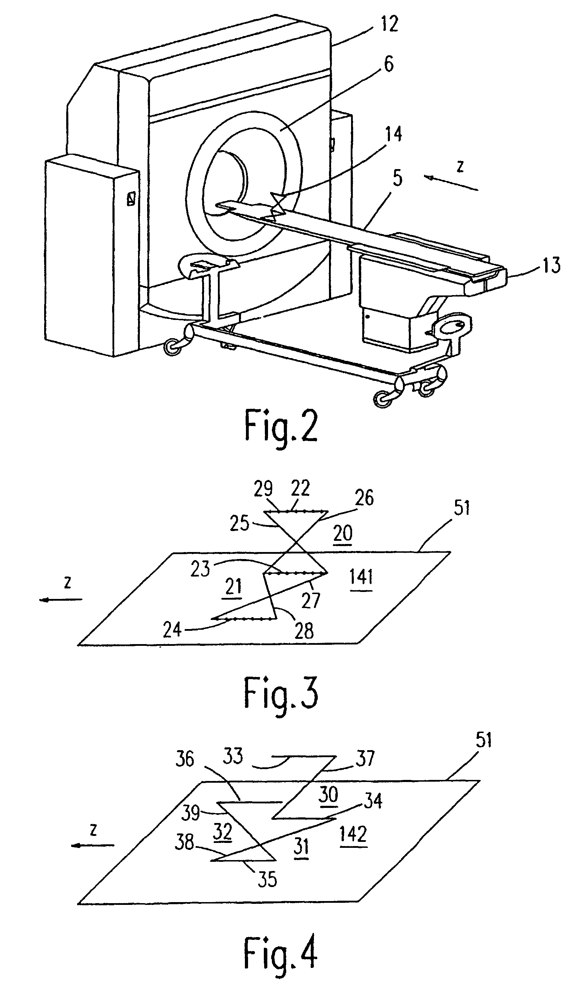

Eine räumliche Darstellung einer CT-Anordnung, bei der die Erfindung verwirklicht werden kann, ist in Fig. 2 gezeigt. Zu erkennen ist die rotierende Scannereinheit 6, die sich in dem Scannergehäuse 12 befindet und hier einen kreisförmigen Querschnitt aufweist. Während der Untersuchung wird der auf dem Patiententisch 5 liegende Patient mittels eines Patiententransportsystems 13 in z-Richtung durch den Untersuchungsbereich innerhalb der Scannereinheit 6 verschoben. Erfindungsgemäß befindet sich gleichzeitig im Durchstrahlungsbereich ein Phantomkörper 14, der ebenfalls durchstrahlt wird und aus dessen Bildpunkten eine Korrekturvorschrift zur Korrektur von Bildverzerrungen ermittelt wird.

Ein erfindungsgemäßer Phantomkörper 141 ist in Fig. 3 gezeigt. Der Phantomkörper 141 (im folgenden X-Rahmen genannt) besteht aus zwei senkrecht zueinander angeordneten X-Elementen 20, 21. Jedes X-Element 20, 21 weist die Kontur einer Sanduhr auf und besteht aus zwei parallelen, in z-Richtung ausgerichteten Stäben 22, 23 bzw. 23, 24 und zwei diagonalen, die Endpunkte der parallelen Stäbe 22, 23, 24 verbindenden Stäben 25, 26, 27, 28. Der Stab 23 ist hier beiden X-Elementen 20 und 21 gemeinsam, es könnten jedoch auch zwei Stäbe, jeweils einer pro X-Element 20, 21 verwendet werden. Das X-Element 21 ist liegt in der Patiententischebene und ist fest mit dem Patiententisch 51 verbunden. Das X-Element 20 liegt senkrecht zur Patiententischebene und ist an einer festen Stelle relativ zum Patiententisch 51 angeordnet. Der X-Rahmen 141 wird so angeordnet, daß er zusammen mit dem zu durchstrahlenden Bereich des Patienten durch den Durchstrahlungsbereich bewegt wird. Wenn beispielsweise der Kopf eines Patienten durchstrahlt werden soll, ist der X-Rahmen 141 so angeordnet, daß sich der Kopf im Bereich der X-Elemente 20,21 befindet. Die Schichtebenen, in denen der Patient durchstrahlt wird und die die Schichtbilder ergeben, liegen idealerweise senkrecht zu den beiden X-Elementen 20, 21, also senkrecht zur z-Richtung, die der Vorschubrichtung des Patiententisches 51 entspricht.

Die einzelnen Stäbe 22 bis 28 des X-Rahmens können auch länger oder kürzer als in Fig. 3 gezeigt sein, d.h., die diagonalen Stäbe 25 bis 28 können beispielsweise über die parallelen Stäbe 22 bis 24 hinausragen oder umgekehrt. Wichtig ist dabei nur, daß von den einzelnen Stäben 22 bis 28 soviele Bildpunkte in verschiedenen Schichtbildern abgebildet werden, daß sich daraus den Stäben entsprechende Lininen in den CT-Bildern rekonstruieren lassen.

Entlang der Stäbe 22 bis 24 sind eine Anzahl von Markierungen 29 z.B. Metallkugeln vorgesehen, die in gleichen Abständen von beispielsweise 2mm angeordnet sind. Die Abstände sind so gewählt, daß in jeder Schicht mindestens eine Markierung 29 pro Stab abgebildet wird. Die Markierungen dienen wie eingangs beschrieben zur Detektion von Veränderungen der Tischvorschubgeschwindigkeit während der Durchstrahlung. Der Genauigkeit halber sind auf drei Stäben 22 bis 24 derartige Markierungen 29 angebracht, es würden aber auch Markierungen nur auf einem der Stäbe 22 bis 24 ausreichen.

Eine weitere Ausführungsform eines Phantomkörpers, der zur Durchführung des erfindungsgemäßen Verfahrens verwendet werden kann, ist in Fig. 4 dargestellt. Der Phantomkörper 142 (im folgenden N-Rahmen genannt) besteht hier aus drei N-Elementen 30, 31, 32, wobei zwei N-Elemente 30, 32 parallel zueinander und senkrecht zum dritten N-Element 31 angeordnet sind. Jedes N-Element 30, 31, 32 besteht aus zwei parallelen, in z-Richtung ausgerichteten Stäben 33, 34 bzw. 34, 35 bzw. 35, 36 und jeweils einem diagonal angeordneten Stab 37, 38, 39. Die N-Elemente 30 und 31 haben den Stab 34, die N-Elemente 31 und 32 den Stab 35 gemeinsam. Das mittlere N-Element 31 ist hier mit dem Patiententisch 51 verbunden. Die N-Elemente 30 bis 32 können beispielsweise zur Untersuchung des Kopfes eines Patienten so dimensioniert sein, daß der Kopf zwischen den N-Elementen 31 und 32 und auf dem N-Element 31 zu liegen kommt. Die Schichtebenen liegen hier idealerweise ebenfalls senkrecht zur z-Richtung.

Sowohl bei dem in Fig. 3 gezeigten X-Rahmen 141 als auch bei dem in Fig. 4 gezeigten N-Rahmen 142 bestehen die Stäbe 22 bis 28 bzw. 33 bis 39 aus Röntgenstrahlung absorbierendem Material. Damit diese Phantomkörper zur Detektion und Korrektur von Bildverzerrungen verwendet werden können, müssen die Phantomkörper mit hoher Präzision hergestellt und mechanisch robust sein. Während der Durchstrahlung darf sich keine Veränderung der mechanischen Abmessungen und der Lage der einzelnen Elemente zueinander ergeben.

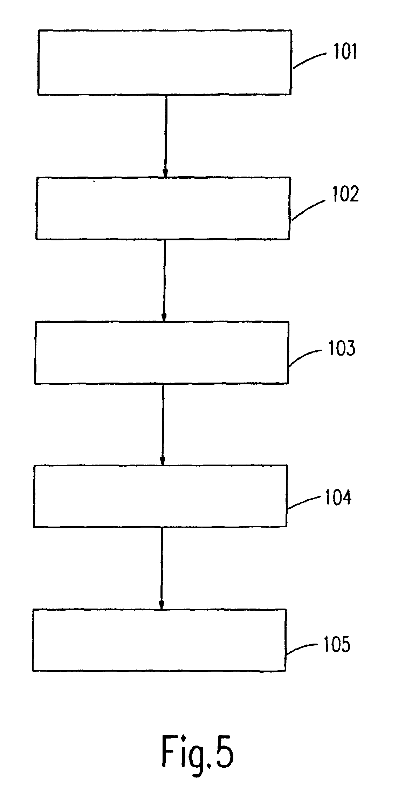

Anhand des in Fig. 5 gezeigten Ablaufplans sollen die zur Durchführung des erfindungsgemäßen Verfahrens nötigen Verfahrensschritte erläutert werden. Im ersten Verfahrensschritt 101 werden zunächst einzelne Schichtbilder des zu untersuchenden Bereichs eines Patienten, beispielsweise des Kopfes, aufgenommen. Dabei befindet sich gleichzeitig auch ein Phantomkörper im Durchstrahlungsbereich, so daß sich in den Schichtbildern auch Bildpunkte des Phantomkörpers zeigen. Zur weiteren Erläuterung sei angenommen, daß sich im Durchstrahlungsbereich während der Durchstrahlung ein in Fig. 3 gezeigter X-Rahmen befand.

Im zweiten Verfahrensschritt 102 werden die Bildpunkte des Phantomkörpers in den Schichtbildern markiert und so miteinander verbunden, daß sich das gesamte Abbild (Phantombild) des Phantomkörpers ergibt. Zur Verdeutlichung dessen sind in Fig. 6 ein einzelnes X-Element 40 und vier Schichtebenen A, B, C, D gezeigt. Die Schnittpunkte (= Bildpunkte) der Schichtebenen A bis D mit dem ersten Stab 41 des X-Elements 40 sind mit A1 bis D1 bezeichnet, die Bildpunkte entlang dem Stab 42 mit A2 bis D2, die Bildpunkte entlang dem Stab 43 mit A3 bis D3 und die Bildpunkte entlang dem Stab 44 mit A4 bis D4. Im allgemeinen ergeben sich mit einem X-Element pro Schichtebene vier Bildpunkte, bei Betrachtung des ganzen in Fig. 3 gezeigten X-Rahmens ergeben sich also insgesamt 7 Bildpunkte pro Schnittebene. Es werden nun in verschiedenen Schichtebenen liegende Bildpunkte durch Verbindungslinien so miteinander verbunden, daß die ursprüngliche Form des Phantomkörpers rekonstruiert wird. Die Verbindungslinien stellen dann die Abbildungen der Stäbe 41 bis 44 dar. Aus den Schnittpunkten jeweils zweier Verbindungslinien ergeben sich die Orte, an denen die Eckpunkte des Phantomkörpers bei einer vollständigen Durchstrahlung des Phantomkörpers liegen würden. Aus den Schnittpunkten der Verbindungslinien 41 und 42 ergibt sich beispielsweise der Eckpunkt E1, aus den Schnittpunkten der Linien 41 und 43 der Eckpunkt E2 usw. Bei einem X-Rahmen gemäß Fig. 3 ergeben sich somit insgesamt sechs Eckpunkte. Aus diesen sechs Eckpunkten läßt sich das Phantombild, also die Abbildung des Phantomkörpers in dem CT-Gesamtbild berechnen.

Im nächsten Verfahrensschritt 103 wird jeder Eckpunkt des Phantombildes dem korrespondierenden (realen) Eckpunkt des Phantomkörpers zugeordnet. Die Transformation eines Bildpunktes in den realen Punkt wird durch die folgende Transformation beschrieben:

$$x_R = t + R\ C\ S\ x_B + b = t + A\ x_B + b.$$

Dabei enthalten der Vektor $x_R$ die Koordinaten des realen Punktes und $x_B$ die Koordinaten des zugehörigen Bildpunktes. Der Vektor t beschreibt eine translatorische Verschiebung zwischen Bild-Koordinatensystem und Patiententisch-Koordinatensystem, die Matrix $R$ eine Rotation zwischen diesen beiden Koordinatensystemen, die Matrix $C$ eine Skalenverzerrung z.B. durch Fehler bei der Tischvorschubgeschwindigkeit und/oder eine durch die Abbildung bedingte Skalenverzerrung in den Schichten selbst, S eine scherungsartige Verzerrung (z.B. durch Fehlwinkeleinstellung der Durchstrahlungsebene) und der Vektor $b$ den Einfluß einer Patiententischdurchbiegung. Die durch die Matrizen $R, C$ und $S$ beschriebenen Verzerrungen können zu einer Matrix $A$ zusammengefaßt werden. Mit dieser Gleichung sind alle eingangs genannten Verzerrungen beschrieben.

Da für die Eckpunkte die Koordinaten der Bildpunkte $x_B$ und der zugehörigen realen Punkte $x_R$ bekannt sind, kann im Verfahrensschritt 104 ein Gleichungssystem aufgestellt werden, mit der alle Unbekannte in obiger Gleichung berechnet werden können. Insgesamt sind dreizehn Unbekannte zu bestimmen, nämlich für die Verschiebung $t$ drei Unbekannte, für die Verzerrungen $A = R\ C\ S$ neun Unbekannte und für die Tischdurchbiegung $b$ eine Unbekannte. Da die Tischdurchbiegung nur in einer Dimension (nämlich in Richtung der Gravitationskraft) auftritt, ist nur ein Element des Vektors $b$ ungleich Null, nämlich die zweite, in Richtung der Gravitationskraft zeigende Vektorkomponente. Der Vektor $b$ läßt sich auch schreiben $b = (0\ b_0 z_B^2\ 0)^T$. Dabei ist mit $z_B$ die dritte, in Vorschubrichtung des Tisches zeigende Vektorkomponente des Bildpunktes $x_B$ gemeint, $b_0$ ist die zu bestimmende Unbekannte.

Es sind also insgesamt dreizehn voneinander unabhängige Gleichungen erforderlich. Durch die Verwendung eines X-Rahmens gemäß Fig. 3, bei dem sechs Eckpunkte mit jeweils drei Raumkoordinaten bestimmt werden, erhält man insgesamt achtzehn Gleichungen. Mit dem in Fig. 4 gezeigten N-Rahmen können ebenfalls sechs Eckpunkte bestimmt werden. Die Berechnung der Unbekannten durch Lösung des beschriebenen Gleichungssystems kann nach herkömmlichen bekannten Methoden erfolgen, was hier nicht näher erläutert werden soll.

Im letzten Verfahrensschritt 105 wird nach Bestimmung aller Unbekannten die Korrekturvorschrift zur Korrektur von Bildverzerrungen aufgestellt und auf alle weiteren Bildpunkte des CT-Gesamtbildes angewendet. Dazu werden in obige Gleichung, die die Korrekturvorschrift darstellt und in der nun die Matrix $A$ und die Vektoren $b$ und $t$ bekannt sind, alle Bildpunkte des CT-Gesamtbildes (oder nur eines besonders interessierenden Ausschnitts oder nur einiger Teilbilder) eingesetzt. Aus den berechneten Bildpunkten wird dann das verzerrungskorrigierte Bild erstellt.

Es kann gegebenenfalls auch eine Berechnung der einzelnen Einflüsse der Verzerrungen durch Zerlegung der Matrix $A$ in die drei Matrizen $R, C$ und $S$ erfolgen. Dazu können bekannte mathematische Verfahren benutzt werden, worauf hier nicht eingegangen werden soll.

Das erfindungsgemäße Verfahren erlaubt während der Bildaufnahme die Berücksichtigung der unmittelbaren Aufnahmesituation. Dadurch wird Bildverzerrungen, beispielsweise aufgrund einer durch das Gewicht des Patienten hervorgerufenen Durchbiegung des Tisches und scherungsartigen Verzerrungen durch ungenaue Kalibrierung des CT-Scanners, Rechnung getragen. Gleichzeitig werden auch Ungenauigkeiten in der Vorschubgeschwindigkeit des Patiententisches erfaßt. Die Erfindung verbessert die Genauigkeit der CT-Bilder, ohne den Patienten einer zusätzlichen Strahlenbelastung auszusetzen. Dadurch wird insbesondere die Genauigkeit neurochirurgischer Eingriffe erheblich erhöht bei Lokalisationssystemen sowohl mit als auch ohne Stereotaxie-Rahmen.

Eine weitere Anwendungsmöglichkeit des erfindungsgemäßen Verfahrens liegt in der Möglichkeit, eine CT-Anordnung vor der Durchstrahlung eines Patienten zu kalibrieren. Dazu wird der Phantomkörper durchstrahlt und das Phantombild ermittelt, und anschließend werden mit Hilfe des Phantombildes und des Phantomkörpers Fehljustierungen der CT-Anordnungen detektiert, die dann vor der Durchstrahlung des Patienten korrigiert werden können. Aufgrund der hohen Genauigkeit dieser Kalibriermethode mit Hilfe des Phantomkörpers können auch geringe Winkel-Fehljustierungen zwischen Patiententischebene und Durchstrahlungsebene im Bereich von 1°-2° korrigiert werden. Für bestimmte Anwendungen ist auch eine Winkeleinstellung zwischen Patiententischebene und Durchstrahlungsebene ungleich 90° gewünscht. Auch bei solchen Winkeleinstellungen kann nach dem beschriebenen Verfahren eine Korrektur von Fehleinstellungen erfolgen.

**Patentansprüche**

1. Verfahren zur Detektion und Korrektur von Bildverzerrungen bei der Röntgen-Computertomographie, bei dem von einem Objekt oder einem Objektbereich mehrere Bilder in verschiedenen Schichtebenen (A,B,C,D) erstellt werden,
   **dadurch gekennzeichnet, daß** während der Erstellung der Bilder des Objektes oder Objektbereiches gleichzeitig ein Phantomkörper (14) im Abbildungsbereich (3) angeordnet ist, der mehrere Elemente (20, 21; 30, 31, 32) enthält, die in verschiedenen, zu den Schichtebenen (A,B,C,D) senkrechten Ebenen liegen,
   und daß aus der Lage der Bildpunkte (A1...A4, B1...B4, C1...C4, D1...D4) des Phantomkörpers (14) in den verschiedenen Bildern des Objekts oder Objektbereichs eine Korrekturvorschrift zur Korrektur der in den verschiedenen Raumrichtungen auftretenden Bildverzerrungen ermittelt wird.

2. Verfahren nach Anspruch 1,
   **dadurch gekennzeichnet, daß** als Phantomkörper (14) ein Rahmen (141, 142) verwendet wird, der mehrere Elemente (20, 21; 30, 31, 32) enthält, die in zu den in den Bildern abgebildeten Ebenen (A,B,C,D) senkrechten und miteinander einen von 0° verschiedenen - votzugsweise 90° betragenden - Winkel einschließenden Ebenen angeordnet sind, und daß der Phantomkörper (14) derart angeordnet wird, daß die Ebenen, in denen die Elemente (20, 21; 30, 31, 32) angeordnet sind, senkrecht zu den in den Bildern abgebildeten Ebenen (A,B,C,D) liegen.

3. Verfahren nach Anspruch 2,
   **dadurch gekennzeichnet, daß** als Phantomkörper (14) ein zwei X-förmige X-Elemente (20, 21) enthaltender X-Rahmen (141) verwendet wird, wobei jedes X-Element die Kontur einer Sanduhr aufweist, und daß der Phantomkörper (14) derart angeordnet wird, daß die Ebenen, in denen die X-Elemente (20,21) angeordnet sind, senkrecht zu den in den Bildern abgebildeten Ebenen (A,B,C,D) liegen.

4. Verfahren nach einem der Ansprüche 1 oder 2,
   **dadurch gekennzeichnet, daß** als Phantomkörper (14) ein drei N-förmige N-Elemente (30, 31, 32) enthaltender N-Rahmen (142) verwendet wird, bei dem zwei N-Elemente (30, 32) parallel zueinander und senkrecht zum dritten N-Element (31) angeordnet sind, und daß der Phantomkörper (14) derart angeordnet wird, daß die Ebenen, in denen die N-Elemente (30,31,32) angeordnet sind, senkrecht zu den in den Bildern abgebildeten Ebenen (A,B,C, D) liegen.

5. Verfahren nach einem der Ansprüche 3 oder 4,
   **dadurch gekennzeichnet, daß** die Bildkoordinaten von mindestens fünf charakteristischen Bildpunkten des Phantomkörpers bestimmt werden und daß aus den Bildkoordinaten und den Koordinaten der korrespondierenden charakteristischen Punkte des Phantomkörpers (14) im Raum die Korrekturvorschrift ermittelt wird

6. Verfahren nach Anspruch 5,
   **dadurch gekennzeichnet, daß** als charakteristische Punkte die Eckpunkte (E1...E4) des N-Rahmens (142) bzw. des X-Rahmens (141) ausgewählt werden.

7. Verfahren nach einem der vorhergehenden Ansprüche,
   **dadurch gekennzeichnet, daß** der Phantomkörper (14) mit einem Patiententisch (5) verbunden ist.

8. Verfahren nach Anspruch 7,
   **dadurch gekennzeichnet, daß** mit Hilfe von am Phantomkörper (14) in zur Verschiebungsrichtung (z) des Patiententisches (5) paralleler Richtung angebrachten Markierungen (29) Veränderungen der Geschwindigkeit des Patiententisches (5) relativ zum Abbildungsbereich (3) während der Erstellung der Bilder detektiert werden.

9. Verfahren nach einem der vorhergehenden Ansprüche,
   **dadurch gekennzeichnet, daß** die Bilder als Schichtbilder durch Computertomographie erstellt werden.

10. Verfahren nach Anspruch 9,
    **dadurch gekennzeichnet, daß** aus mehreren Schichtbildern ein CT-Bild und aus den Bildpunkten (A1...A4, B1...B4, C1...C4, D1...D4) des Phantomkörpers (14) in mehreren Schichtbildern ein Phantombild des Phantomkörpers (14) ermittelt wird.

11. Verfahren nach Anspruch 10,
    **dadurch gekennzeichnet, daß** die Korrekturvorschrift durch Einsetzen einer Anzahl *von* Bildpunkten ($x_B$) des Phantomkörpers (14) im Phantombild und den korrespondierenden Punkten ($x_R$) des Phantomkörpers (14) im Raum in eine zwischen Bildpunkten und Punkten im Raum ($x_R$) bestehende lineare Gleichung ermittelt wird und daß danach eine Korrektur der Bildverzerrungen durch Einsetzen weiterer Bildpunkte ($x_B$) des CT-Bildes in diese Gleichung erfolgen kann.

12. CT-Anordnung zur Durchführung des Verfahrens nach Anspruch 1 mit einer Röntgendetektoranordnung (7) und einer Recheneinheit (9) zur Verarbeitung gemessener Signale und zur Berechnung von Schichtbildern aus den gemessenen Signalen,

**dadurch gekennzeichnet, daß** die Recheneinheit (9) so gestaltet ist, daß sie aus verschiedenen Bildern eines Objekts oder eines Objektbereichs in verschiedenen Schichtebenen (A,B,C,D) und eines während der Erstellung der Bilder im Abbildungsbereich (3) gleichzeitig angeordneten Phantomkörpers (14), der mehrere Elemente (20, 21; 30, 31, 32) enthält, die in verschiedenen, zu den Schichtebenen (A,B,C,D) senkrechten Ebenen liegen, aus der Lage der Bildpunkte (A1...A4, B1...B4, C1...C4, D1...D4) des Phantomkörpers (14) in den verschiedenen Bildern des Objekts oder Objektbereichs eine Korrekturvorschrift zur Korrektur der in den verschiedenen Raumrichtungen auftretenden Bildverzerrungen ermittelt.

**13.** CT-Anordnung nach Anspruch 12,
**dadurch gekennzeichnet, daß** als Phantomkörper (14) ein zwei X-förmige X-Elemente (20,21) enthaltender X-Rahmen (141) oder ein drei N-förmige N-Elemente (30,31,32) enthaltender N-Rahmen (142), bei dem zwei N-Elemente (30,32) parallel zueinander und senkrecht zum dritten N-Element (31) angeordnet sind, verwendet wird, und daß der Phantomkörper (14) derart angeordnet wird, daß die Ebenen, in denen die X-Elemente (20,21) bzw. die N-Elemente (30,31,32) angeordnet sind, senkrecht zu den Schichtebenen (A,B,C,D) der Schichtbilder liegen.

## Claims

**1.** A method for the detection and correction of image distortions in X-ray computed tomography in which a plurality of images of an object or object zone are formed in different slice planes (A,B,C,D), **characterized in that** during the formation of the images of the object or the object zone a phantom body (14) is arranged in the imaging zone (3), which phantom body comprises a plurality of elements (20,21; 30,31,32) which are situated in different planes extending perpendicularly to the slice planes (A,B,C, D), and that a correction rule for the correction of the image distortions occurring in the various spatial directions is derived from the position of the image points (A1...A4, B1...B4, C1...C4, D1...D4) of the phantom body (14) in the various images of the object or the object zone.

**2.** A method as claimed in Claim 1, **characterized in that** use is made of a phantom body (14) in the form of a frame (141,142) with a plurality of elements (20,21; 30,31,32) which are arranged in planes which extend perpendicularly to the planes (A,B,C, D) imaged in the images and enclose an angle other than 0°, preferably 90°, relative to one another, and that the phantom body (14) is arranged so that the planes in which the elements (20,21; 30,31,32) are situated extend perpendicularly to the planes (A,B, C,D) images in the images.

**3.** A method as claimed in claim 2, **characterized in that** use is made of a phantom body (14) in the form of an X-frame (141) with two X-shaped X-elements (20,21), each having the contour of an hour glass, the phantom body (14) being arranged in such a manner that the planes in which the X-elements (20,21) are arranged extend perpendicularly to the planes (A,B,C,D) imaged in the images.

**4.** A method as claimed in one of the Claims 1 or 2, **characterized in that** use is made of a phantom body (14) in the form of an N-frame (142) with three N-shaped N-elements (30, 31, 32), two N-elements (30, 32) extending parallel to one another and perpendicularly to the third N-element (31), the phantom body (14) being arranged in such a manner that the planes in which the N-elements (30, 31, *32)* are arranged extend perpendicularly to the planes (A, B, C, D) imaged in the images.

**5.** A method as claimed in one of the Claims 3 or 4, **characterized in that** the image coordinates of at least five characteristic image points of the phantom body are determined and that the correction rule is determined from the image coordinates and the coordinates of the corresponding characteristic points of the phantom body (14) in space.

**6.** A method as claimed in Claim 5, **characterized in that** the corner points (E1 ... E4) of the N-frame (142) or the X-frame (141) are chosen as the characteristic points.

**7.** A method as claimed in one of the preceding Claims, **characterized in that** the phantom body (14) is connected to a patient table (5).

**8.** A method as claimed in Claim 7, **characterized in that** marks (29) are provided on the phantom body (14) in the direction parallel to the displacement direction (z) of the patient table (5) in order to detect changes of the speed of the patient table (5) relative to the imaging zone (3) during the formation of the images.

**9.** A method as claimed in one of the preceding Claims,
**characterized in that** the images are formed as slice images by computed tomography.

**10.** A method as claimed in Claim 9, **characterized in that** a CT image is formed from a plurality of slice images and a phantom image of the phantom body (14) is determined from the image points (A1...A4, B1...B4, C1...C4, D1...D4) of the phantom body (14) in a plurality of slice images.

**11.** A method as claimed in Claim 10, **characterized in that** the correction rule is determined by inserting a number of image points ($X_B$) of the phantom body (14) in the phantom image and the corresponding points ($X_R$) of the phantom body (14) in space in a linear equation, existing between image points and points in space ($X_R$), after which the image distortions can be corrected by inserting further image points ($X_B$) of the CT image in said equation.

**12.** A CT apparatus for carrying out the method claimed in claim 1, which apparatus includes an X-ray detector arrangement (7) and an arithmetic unit (9) for processing measured signals and for calculating slice images from the measured signals, **characterized in that** the arithmetic unit (9) is constructed so that it determines a correction rule for the correction of the image distortions occurring in the various spatial directions from different images of an object or an object zone in different slice planes (A, B, C, D) and of a phantom body (14) which is also arranged in the imaging zone (3) during the formation of the images and which comprises a plurality of elements (20,21; 30,31,32) which are situated in different planes, extending perpendicularly to the slice planes (A,B,C,D) from the position of the image points (A1...A4, B1...B4, C1...C4, D1...D4) of the phantom body (14) in the various images.

**13.** A CT apparatus as claimed in Claim 12, **characterized in that** use is made of a phantom body (14) in the form of an X-frame (141) with two X-shaped X-elements (20, 21) or of an N-frame (142) with three N-shaped N-elements (30, 31, 32), two N-elements (30, 32) extending parallel to one another and perpendicularly to the third N-element (31), the phantom body (14) being arranged in such a manner that the planes in which the X-elements (20, 21) or the N-elements (30, 31, 32) are arranged extend perpendicularly to the slice planes (A, B, C, D) ofthe slice images.

**Revendications**

**1.** Procédé de détection et de correction de distorsions d'images en tomographie informatisée, plusieurs images d'un objet ou d'une zone de l'objet étant produites dans différents plans de coupe (A, B,C,D),
**caractérisé en ce que**, pendant la production des images de l'objet ou de la zone de l'objet, un corps fantôme (14) est simultanément disposé dans la zone de représentation (3) et contient plusieurs éléments (20,21;30,31,32) qui sont disposés dans différents plans perpendiculaires aux plans de coupe (A,B,C,D) et que, à partir de la position des points d'image (A1...A4, B1...B4, C1...C4, D1...D4) du corps fantôme (14) dans les différentes images de l'objet ou de la zone de l'objet, une prescription de correction est déterminée en vue de la correction des distorsions d'image se produisant dans les différentes directions spatiales.

**2.** Procédé selon la revendication 1,
**caractérisé en ce que** le corps fantôme (14) utilisé est un cadre (141,142) qui contient plusieurs éléments (20,21;30,31,32) qui sont disposés dans des plans perpendiculaires aux plans (A,B,C,D) représentés dans les images et formant entre eux un angle différent de 0° - de préférence de 90° et que le corps fantôme (14) est disposé de telle sorte que les plans dans lesquels sont disposés les éléments (20,21;30,31,32) sont perpendiculaires aux plans (A,B,C,D) représentés dans les images.

**3.** Procédé selon la revendication 2,
**caractérisé en ce que** le corps fantôme (14) utilisé est un cadre X (141) contenant deux éléments X (20,21) en forme de X, chaque élément C présentant le contour d'un sablier et que le corps fantôme (14) est disposé de telle sorte que les plans dans lesquels sont disposés les éléments X (20,21) sont perpendiculaires aux plans (A,B,C,D) représentés dans les images.

**4.** Procédé selon l'une des revendications 1 ou 2,
**caractérisé en ce qu'**un cadre N (142) contenant trois éléments N (30,31,32) en forme de N est utilisé, deux éléments N (30,32) étant parallèles l'un à l'autre et perpendiculaires au troisième élément N (31) et que le corps fantôme (14) est disposé de telle sorte que les plans dans lesquels sont disposés les éléments N (30,31,32) sont perpendiculaires aux plans (A,B,C,D) représentés dans les images.

**5.** Procédé selon l'une des revendications 3 ou 4,
**caractérisé en ce que** les coordonnées de l'image sont déterminées par au moins cinq points d'image caractéristiques du corps fantôme et que la prescription de correction est déterminée à partir des coordonnées de l'image et des coordonnées des points caractéristiques correspondants du corps fantôme (14) dans l'espace.

**6.** Procédé selon la revendication 5,
**caractérisé en ce que** les points de coin (E1...E4) du cadre N (142) ou du cadre X (141) ont été sélectionnés comme points caractéristiques.

**7.** Procédé selon l'une des revendications précédentes,

**caractérisé en ce que** le corps fantôme (14) est fixé à une table de patient (5).

8. Procédé selon la revendication 7,
   **caractérisé en ce que** des repères (29) appliqués sur le corps fantôme (14) dans une direction parallèle au sens de coulissement (z) de la table de patient (5) permettent de détecter des changements de la vitesse de la table de patient (5) par rapport à la zone de représentation (3) pendant la réalisation des clichés.

9. Procédé selon l'une des revendications précédentes,
   **caractérisé en ce que** les clichés sont réalisés comme des clichés en couche par tomographie informatisée.

10. Procédé selon la revendication 9,
    **caractérisé en ce qu'**un cliché CT est déterminé à partir de plusieurs clichés en couche et un cliché fantôme du corps fantôme (14) est déterminé à partir des points d'image (A1...A4, B1...B4, C1...C4, D1...D4) du corps fantôme (14) en plusieurs clichés en couches.

11. Procédé selon la revendication 10,
    **caractérisé en ce que** la prescription de correction est déterminée par l'utilisation de plusieurs points d'image ($x_B$) du corps fantôme (14) dans le cliché fantôme et les points correspondants ($x_R$) du corps fantôme (14) dans l'espace en une équation linéaire existant entre les points d'image et les points dans l'espace ($x_R$) et qu'ensuite, une correction des distorsions d'image peut être effectuée par utilisation d'autres points d'image ($x_B$) de l'image CT dans cette équation.

12. Dispositif de CT pour la mise en oeuvre du procédé selon la revendication 1 avec un dispositif détecteur de rayons X (7) et une unité de calcul (9) pour le traitement de signaux mesurés et pour le calcul d'images en couches à partir des signaux mesurés, **caractérisé en ce que** l'unité de calcul (9) est conçue de telle sorte que, pour la correction des distorsions d'image se produisant dans les différentes directions de l'espace, elle détermine une prescription de correction à partir des différentes images d'un objet ou d'une zone de l'objet dans différents plans de coupe (A,B,C,D) et d'un corps fantôme (14) disposé simultanément pendant la réalisation des clichés dans la zone de représentation (3) qui contient plusieurs éléments (20,21;30,31,32) situés dans différents plans perpendiculaires aux plans de coupe (A,B,C,D) à partir de la position des points d'image (A1...A4,B1...B4,C1...C4,D1...D4) du corps fantôme (14) dans les différents clichés de l'objet ou de la zone de l'objet.

13. Dispositif CT selon la revendication 12,
    **caractérisé en ce que** le corps fantôme utilisé (14) est un cadre X (141) contenant deux éléments X (20,21) en forme de X ou un cadre N (142) contenant trois éléments N (30,31,32) en forme de N, deux éléments N (30,32) étant disposés parallèlement l'un à l'autre et perpendiculairement au troisième élément N (31) et que le corps fantôme (14) est disposé de telle sorte que les plans dans lesquels sont disposés les éléments X (20,21) ou les éléments N (30,31,32) sont situés perpendiculairement aux plans de couche (A,B,C,D) des clichés en couche.

Fig.1

Fig.6

Fig.2

Fig.3

Fig.4

Fig.5